(19) Europäisches Patentamt
European Patent Office
Office européen des brevets

(11) EP 2 604 615 A1

(12) EUROPÄISCHE PATENTANMELDUNG

(43) Veröffentlichungstag:
**19.06.2013 Patentblatt 2013/25**

(21) Anmeldenummer: **11193045.9**

(22) Anmeldetag: **12.12.2011**

(51) Int Cl.:
***C07F 9/94*** *(2006.01)*
***C07C 235/74*** *(2006.01)*
***C07C 235/82*** *(2006.01)*
***C08G 18/12*** *(2006.01)*
***C09D 175/04*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Sika Technology AG**
**6340 Baar (CH)**

(72) Erfinder:
- **Cannas, Rita**
**8600 Dübendorf (CH)**
- **Burckhardt, Urs**
**8049 Zürich (CH)**

(74) Vertreter: **Sika Patent Attorneys**
**c/o Sika Technology AG**
**Tüffenwies 16-22**
**8048 Zürich (CH)**

# (54) Bismuthaltiger Katalysator für Polyurethan-Zusammensetzungen

(57) Die vorliegende Erfindung betrifft bismuthaltige Katalysatoren, erhältlich durch die Umsetzung von mindestens einem Bismut(III)-Salz oder Bismut(III)-Komplex mit mindestens einem 1,3-Ketoamid der Formel (I). Derartige Komplexverbindungen sind insbesondere als Katalysator für ein- und zweikomponentige Polyurethan-Zusammensetzungen geeignet. Die Erfindung betrifft auch zweikomponentige Polyurethan-Zusammensetzungen, umfassend mindestens ein Polyisocyanat als erste Komponente, mindestens ein Polyol als zweite Komponente und mindestens einen derartigen bismuthaltigen Katalysator. Die Erfindung betrifft ferner einkomponentige Polyurethan-Zusammensetzungen, umfassend mindestens ein Polyurethanprepolymer mit Isocyanatgruppen, hergestellt aus mindestens einem Polyisocyanat mit mindestens einem Polyol, und einem derartigen bismuthaltigen Katalysator. Zusätzlich betrifft die Erfindung verschiedene Verwendungen der genannten Polyurethan-Zusammensetzungen.

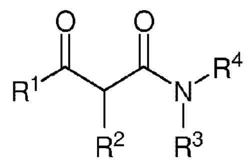

(I)

EP 2 604 615 A1

## Beschreibung

### Technisches Gebiet

Die vorliegende Erfindung betrifft das Gebiet der Polyurethan-Zusammensetzungen sowie Katalysatoren für Polyurethan-Zusammensetzungen.

### Stand der Technik

Polyurethan-Zusammensetzungen sind seit langem bekannt und werden in vielen Bereichen eingesetzt. Klassisch wird in der Fachwelt zwischen einkomponentigen und zweikomponentigen Polyurethan-Zusammensetzungen unterschieden. Einkomponentige Polyurethan-Zusammensetzungen härten unter dem Einfluss von Luftfeuchtigkeit aus. Zweikomponentige Polyurethan-Zusammensetzungen enthalten als zweite Komponente eine Härterkomponente, welche im Wesentlichen Polyamine und/oder Polyole enthält. In beiden Fällen werden Isocyanatgruppen-haltige Verbindungen oder Prepolymere eingesetzt.

Um die Aushärtung zu beschleunigen, werden Katalysatoren beigefügt. Zwar ist eine Vielzahl von Polyurethan-Katalysatoren bekannt, allerdings ist eine Großzahl hinsichtlich der Urethanisierungsreaktion, d.h. der Reaktion von alkoholischen OH-Gruppen mit Isocyanatgruppen, nicht sonderlich selektiv, sondern katalysiert mehr oder weniger auch andere Reaktionen der Isocyanatgruppe, wie Allophanat- und Biuret-Bildung oder Cyclotrimerisierung. Insbesondere steht die Urethanisierungsreaktion meist in Konkurrenz zur Reaktion der Isocyanatgruppen mit Wasser, welche unter Freisetzung von gasförmigem Kohlendioxid zu Harnstoffgruppen führt. Diese Nebenreaktion ist bei vielen Polyurethan-Zusammensetzungen, insbesondere bei deren Anwendung als Kleb- und Dichtstoff, als Beschichtung oder Gießharz, störend, da sie bei der Aushärtung zu Blasenbildung und damit schlechter Formstabilität, geringerer Haftung, tieferer mechanischer Festigkeit, unbefriedigender Ästhetik sowie zu wenig reproduzierbaren Ergebnissen führt. Das für die Blasenbildung verantwortliche Wasser stammt entweder aus dem Restwasser-Gehalt der Bestandteile der Zusammensetzung, insbesondere der Polyole und der Füllstoffe, welche auch nach Trocknungsprozessen mehr oder weniger feucht sind und einen typischen Restwasser-Gehalt von 0,01 bis 0,5 Gew.-% aufweisen, oder aus der Umgebungsfeuchtigkeit, welche durch Diffusion aus der Luft oder aus den Substraten in die Zusammensetzung eindringt, was besonders bei hoher Luftfeuchtigkeit, porösen Substraten und/oder hydrophilen Polyolen, wie den in der Praxis oft eingesetzten Polyetherpolyolen, auftritt. Gerade die in der Praxis vielfach verwendeten Aminkatalysatoren, beispielsweise tertiäre Amine, und Zinnkatalysatoren, beispielsweise Dialkylzinncarboxylate, führen oft zu ausgeprägter Blasenbildung. Der Restwasser-Gehalt in der Polyurethan-Zusammensetzung bewirkt außerdem, dass hydrolyseempfindliche Katalysatoren, wie beispielsweise Bismutcarboxylate, bei längerem Aufbewahren der Zusammensetzung vor dem Gebrauch (Lagerung) deaktiviert werden, was sich negativ auf die Aushärtungsgeschwindigkeit und die mechanischen Eigenschaften auswirkt. Bei einigen bekannten Katalysatoren, beispielsweise den Dialkylzinncarboxylaten, ist darüber hinaus die Beständigkeit der ausgehärteten Zusammensetzung unter thermischer Belastung ungenügend, wobei der Katalysator einen Molekulargewichts-Abbau, d.h. eine Depolymerisation, unter Verlust an mechanischer Festigkeit verursacht. Weiterhin sind viele der bekannten Katalysatoren bei Raumtemperatur fest und in den Polyurethan-Ausgangsmaterialien oder den Weichmachern wenig löslich, so dass für ihren Einsatz in Zusammensetzungen, die bei Raumtemperatur härten, organische Lösungsmittel eingesetzt werden müssen. Schließlich sind manche der bekannten Katalysatoren, insbesondere solche auf Basis von Schwermetallverbindungen, toxikologisch bedenklich.

Die Verwendung von Bismutverbindungen als Katalysatoren für härtbare Massen, beispielsweise Polyurethan-Zusammensetzungen, ist bekannt. So beschreibt die US-A-4,584,362 den Einsatz von Bismut(III)-tricarboxylaten, wie Bismut-2-ethylhexanoat oder Bismutneodecanoat. Derartige Bismut(III)-tricarboxylate zeichnen sich zwar durch eine sehr hohe katalytische Aktivität bei guter Selektivität in Bezug auf die Urethanisierungsreaktion aus und sind darüber hinaus toxikologisch wenig bedenklich, jedoch sind sie äußerst feuchtigkeitsempfindlich und werden daher bei Lagerung rasch deaktiviert. Gemäß der WO 2004/033519 und der US-A-5,719,229 wurde zwar versucht, Bismut(III)-tricarboxylate mittels Liganden, wie beispielsweise Chinolinen, Carbonsäuren oder Diketonen, zu stabilisieren und/oder die Topfzeit der Polyurethan-Zusammensetzung zu verlängern, die hierbei eingesetzten Liganden bewirken jedoch entweder keine ausreichende Stabilisierung des Bismut(III)-tricarboxylats oder vermindern dessen katalytische Aktivität stark. Der Einsatz von 8-Hydroxychinolin führt außerdem zu einer schlechteren Löslichkeit des Katalysators, was Ausfällungen bewirken und den Einsatz organischer Lösungsmittel erforderlich machen kann. Ferner kommt es zu starken Verfärbungen des Katalysators sowie der ausgehärteten Poly-urethan-Zusammensetzungen, welche unter Lichteinfluss besonders ausgeprägt sind.

### Darstellung der Erfindung

Aufgabe der vorliegenden Erfindung ist es, die vorstehend beschriebenen Nachteile des Standes der Technik

zu beseitigen. Insbesondere besteht die Aufgabe der vorliegenden Erfindung darin, einen Katalysator bereitzustellen, der zu einer Verbesserung nachfolgender Eigenschaften bzw. zu einem ausgewogenen Verhältnis dieser führt.

Der Katalysator soll sich durch eine hohe katalytische Aktivität und Selektivität hinsichtlich der Urethanisierungsreaktion, d.h. der Reaktion von alkoholischen OH-Gruppen mit Isocyanatgruppen, auszeichnen und so einen raschen und durch Feuchtigkeit möglichst wenig gestörten Aufbau eines mechanisch hochwertigen Polyurethan-Polymers aus polyfunktionellen Alkoholen (Polyolen) und Polyisocyanaten ermöglichen. Zum anderen soll der Katalysator eine ausreichende Hydrolyseresistenz besitzen, um unter üblichen Lagerbedingungen, d.h. bei Raumtemperatur oder bei leicht erhöhten Temperaturen, über mehrere Monate in einer restwasserhaltigen Polyol-Zusammensetzung ohne starken Aktivitätsverlust erhalten zu bleiben. Weiterhin soll der Katalysator die thermische Beständigkeit des ausgehärteten Polyurethan-Polymers möglichst wenig herabsetzen. Überdies soll der Katalysator bei Raumtemperatur oder bei leicht erhöhten Temperaturen flüssig sein bzw. in den Polyurethan-Ausgangsmaterialien oder in Weichmachern gut löslich sein, damit er in bei Raumtemperatur härtenden, lösungsmittelfreien Systemen auf einfache Weise eingesetzt werden kann. Letztendlich soll der Katalysator eine möglichst geringe Giftigkeit aufweisen.

Insbesondere soll der Katalysator über gute thermische und hydrolytische Stabilität verfügen und damit in einem restwasserhaltigen Polyol nicht schnell hydrolysieren und so die katalytische Aktivität auch bei längerer Lagerung bewahren, als auch bei Raumtemperatur flüssig sein und/oder eine gute Löslichkeit in Weichmachern oder Polyolen aufweisen, um damit in bei Raumtemperatur härtenden Systemen auf einfache Weise und ohne die Verwendung von flüchtigen organischen Lösungsmitteln (VOC) eingesetzt werden zu können.

Schließlich soll der Katalysator möglichst farblos sein und sich auch unter Lichteinfluss nicht verfärben, damit dessen Einsatz auch in unpigmentierten oder hellfarbigen Produkten ohne Beeinträchtigungen möglich ist.

Überraschenderweise wurde nun ein bismuthaltiger Katalysator gemäß Anspruch 1 mit den gewünschten Eigenschaften gefunden. Dieser bismuthaltige Katalysator ist durch Umsetzung von mindestens einem Bismut(III)-Salz oder Bismut(III)-Komplex mit mindestens einem 1,3-Ketoamid der Formel (I) erhältlich,

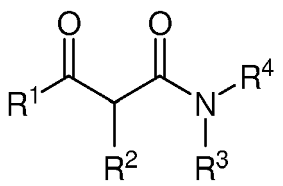

(I),

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und

$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

Das stöchiometrische Verhältnis zwischen dem Bismut(III)-Salz oder Bismut(III)-Komplex und dem 1,3-Ketoamid der Formel (I) liegt vorzugsweise im Bereich von etwa 1:0,5 bis 1:20, besonders bevorzugt im Bereich von 1:1 bis 1:10, und ganz besonders bevorzugt im Bereich von 1:3 bis 1:6. Dies führt zu dem Vorteil, dass der Katalysator eine gute Hydrolysestabilität und gleichzeitig eine hohe katalytische Aktivität aufweist.

Es wird vermutet, dass das Bismut(III)-Salz oder der Bismut(III)-Komplex mit dem 1,3-Ketoamid der Formel (I) eine Komplexbildungsreaktion unter Bildung von Bismut(III)-1,3-Ketoamidat-Komplexen eingeht, in denen mindestens ein 1,3-Ketoamid der Formel (I) formal in einfach negativ geladener Form als Ligand vorliegt.

Da die negative Ladung über die 1,3-Ketoamid-Struktur delokalisiert ist, kann der Ligand in verschiedenen Grenzstrukturen, beispielsweise in den nachfolgend dargestellten Grenzstrukturen, gezeichnet werden. Alle möglichen Grenzstrukturen des Liganden L der Formel (I) werden im Rahmen der vorliegenden Erfindung als gleichwertig angesehen.

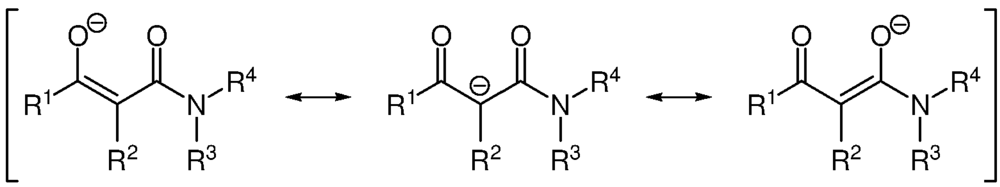

In der Formel (I) stehen $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen.

Bei dem einwertigen gesättigten Kohlenwasserstoff-Rest mit 1 bis 10 Kohlenstoff-Atomen handelt es sich bevorzugt um einen Alkyl-Rest mit 1 bis 4 Kohlenstoffatomen, insbesondere einen Methyl- oder einen Butyl-Rest. Diese haben den Vorteil, dass die Komplexverbindung damit tendenziell flüssig oder gut löslich ist. Bei dem einwertigen ungesättigten Kohlenwasserstoff-Rest handelt es sich bevorzugt auch um einen Arylrest, insbesondere um einen Phenyl-Rest.

Besonders bevorzugt ist $R^2$ ein Wasserstoff-Rest, da die Komplexverbindung damit tendenziell besonders stabil ist.

Unter einem zweiwertigen Alkylen-Rest mit 3 bis 6 Kohlenstoffatomen wird ein Rest der Formel $-(CH_2)_n-$ verstanden, wobei n 3 bis 6 bedeutet.

Bevorzugt bilden $R^1$ und $R^2$ zusammen einen zweiwertigen Alkylen-Rest mit 3 bis 4 Kohlenstoffatomen, insbesondere mit 3 Kohlenstoffatomen.

$R^3$ und $R^4$ stehen unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen.

Bei dem einwertigen gesättigten Kohlenwasserstoff-Rest mit 1 bis 12 Kohlenstoffatomen handelt es sich bevorzugt um einen Alkylrest mit 1 bis 8 Kohlenstoffatomen, besonders bevorzugt um einen Methyl-, einen Ethyl-, einen Propyl-, einen Isopropyl-, einen Butyl-, einen Isobutyl-, einen Hexyl-, einen 2-Methyl-pentyl-, einen Octyl-, oder einen 2-Ethyl-hexyl-Rest. Dies hat den Vorteil, dass die Komplexverbindung damit tendenziell flüssig oder gut löslich ist. Vorzugsweise kann der einwertige gesättigte Kohlenwasserstoff-Rest mit 1 bis 12 Kohlenstoffatomen auch ein Cycloalkyl-Rest mit 5 bis 6 Kohlenstoffatomen, besonders bevorzugt 6 Kohlenstoffatomen, sein. Bei dem einwertigen gesättigten Kohlenwasserstoff-Rest mit Heteroatomen handelt es sich vorzugsweise um einen Hydroxyalkyl-Rest mit 1 bis 4 Kohlenstoffatomen, besonders bevorzugt um einen 2-Hydroxyethyl- oder 2-Hydroxy-propyl-Rest. Dies hat den Vorteil, dass die Komplexverbindung damit tendenziell flüssig oder gut löslich ist und der Ligand bei der Aushärtung in das Polymer kovalent eingebunden werden kann. Bevorzugt ist auch ein Alkylether-Rest mit 1 bis 4 Kohlenstoffatomen, besonders bevorzugt ein 2-Methoxyethyl- oder ein 2-(2-Methoxy)ethoxyethyl-Rest, da die Komplexverbindung damit tendenziell flüssig oder gut löslich ist.

$R^3$ kann zusammen mit $R^4$ vorzugsweise auch einen zweiwertigen Alkylen-Rest der Formel $-(CH_2)_n-X-(CH_2)_n-$ mit X=O, NR, wobei R ein einwertiger Alkyl-Rest mit 1 bis 4 Kohlenstoffatomen ist, oder S und n = 2 bis 4 bilden. Besonders bevorzugt ist n=2 und X=O oder NR.

Die Auswahl der bevorzugten Reste in den Liganden L der Formel (I) beruht vorzugsweise darauf, dass die entsprechenden 1,3-Ketoamide, welche als Ausgangsstoffe zur Herstellung des erfindungsgemäßen bismuthaltigen Katalysators eingesetzt werden, einfach herstellbar und/oder kommerziell erhältlich und damit preisgünstig sind.

Besonders bevorzugt sind nachfolgende bismuthaltige Katalysatoren (1) bis (8) mit Liganden L der Formel (I), wobei $R^1$ bis $R^4$ die in der Tabelle genannten Bedeutungen besitzen.

<table>
<tr><th></th><th>R<sup>1</sup></th><th>R<sup>2</sup></th><th>R<sup>3</sup></th><th>R<sup>4</sup></th></tr>
<tr><td>(1)</td><td>Alkyl-Rest mit 1-4 Kohlenstoffatomen</td><td>Wasserstoff-Rest</td><td>Alkyl-Rest mit 1-8 Kohlenstoffatomen</td><td>Alkyl-Rest mit 1-8-Kohlenstoffatomen</td></tr>
<tr><td>(2)</td><td>Phenyl-Rest</td><td>Wasserstoff-Rest</td><td>Alkyl-Rest mit 1-8 Kohlenstoffatomen</td><td>Alkyl-Rest mit 1-8-Kohlenstoffatomen</td></tr>
<tr><td>(3)</td><td>Alkyl-Rest mit 1-4 Kohlenstoffatomen</td><td>Wasserstoff-Rest</td><td>Alkylether-Rest mit 1-4 Kohlenstoffatomen</td><td>Alkylether-Rest mit 1-4 Kohlenstoffatomen</td></tr>
<tr><td>(4)</td><td colspan="2">Alkylen-Rest mit 3-6 Kohlenstoffatomen</td><td colspan="2">Alkyl-Rest mit 1-8 Kohlenstoffatomen</td></tr>
<tr><td>(5)</td><td>Alkyl-Rest mit 1-4 Kohlenstoffatomen</td><td>Wasserstoff-Rest</td><td colspan="2">Alkylen-Rest der Formel $-(CH_2)_n-X-(CH_2)_n-$ mit X=O oder NR und n=2</td></tr>
<tr><td>(6)</td><td>Alkyl-Rest mit 1-4 Kohlenstoffatomen</td><td>Wasserstoff-Rest</td><td>Cycloalkyl-Rest mit 5-6 Kohlenstoffatomen</td><td>Alkyl-Rest mit 1-8 Kohlenstoffatomen</td></tr>
<tr><td>(7)</td><td>Alkyl-Rest mit 1-4 Kohlenstoffatomen</td><td>Wasserstoff-Rest</td><td>Alkyl-Rest mit 1-8 Kohlenstoffatomen</td><td>Cycloalkyl-Rest mit 5-6 Kohlenstoffatomen</td></tr>
<tr><td>(8)</td><td>Phenyl-Rest</td><td>Wasserstoff-Rest</td><td colspan="2">Alkylen-Rest der Formel $(-CH_2)_n-X-(CH_2)_n-$ mit X=O oder NR und n=2</td></tr>
</table>

Der erfindungsgemäße bismuthaltige Katalysator besitzt vorzugsweise die Formel $Bi(L)_x(Y)_{3-x}$, in der x für 1,2 oder 3 steht, y für einen einfach negativen Liganden steht und L für einen Liganden der Formel (I) steht.

Die Herstellung des bismuthaltigen Katalysators erfolgt durch Umsetzung von mindestens einem Bismut(III)-Salz oder Bismut(III)-Komplex mit mindestens einem 1,3-Ketoamid der Formel (I)

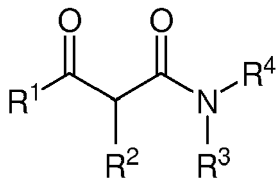

(I)

mit $R^1$, $R^2$, $R^3$ und $R^4$, wie vorstehend definiert. Bevorzugt ist der Einsatz eines Bismut(III)-carboxylates, insbesondere Bismut(III)-neodecanoat oder Bismut(III)-2-ethylhexanoat.

Das mindestens eine Bismut(III)-Salz oder der mindestens eine Bismut(III)-Komplex und das mindestens eine 1,3-Ketoamid der Formel (I) werden vermischt und die Mischung vorzugsweise unter Rühren während 1 bis 24 Stunden, bevorzugt etwa 2 Stunden, auf eine Temperatur von 50 bis 130°C, bevorzugt von etwa 80°C, erwärmt. Bevorzugt wird das Reaktionsgemisch anschließend auf vorzugsweise Raumtemperatur abgekühlt.

Der erfindungsgemäße bismuthaltige Katalysator kann als Katalysator für härtbare Massen, bevorzugt für Polyurethan-Zusammensetzungen, verwendet werden. Der erfindungsgemäße bismuthaltige Katalysator beschleunigt die Aushärtung von härtbaren Massen, welche zu Vernetzungsreaktionen fähige Reaktivgruppen aufweisen. Die härtbaren Massen können einkomponentig und mehrkomponentig formuliert sein.

Bevorzugt beschleunigt der erfindungsgemäße bismuthaltige Katalysator die Aushärtung von zweikomponentigen Polyurethan-Zusammensetzungen, welche mit sich selbst und gegebenenfalls unter dem Einfluss von Feuchtigkeit über blockierte oder insbesondere freie Isocyanatgruppen vernetzen. Dabei wird vor allem die Urethanisierungsreaktion, d.h. die Reaktion von Isocyanatgruppen mit alkoholischen OH-Gruppen, beschleunigt. Die zu vernetzenden Zusammensetzungen können auch weitere zu Vernetzungsreaktionen fähige Reaktivgruppen, wie insbesondere Alkoxysilangruppen, enthalten. Vorzugsweise handelt es sich dabei um Trialkoxysilan-Gruppen, wie sie beispielsweise in Silan-Haftmitteln enthalten sind.

Der erfindungsgemäße bismuthaltige Katalysator kann vorteilhafterweise als Katalysator in einer zweikomponentigen Polyurethan-Zusammensetzung eingesetzt werden. Diese umfasst neben dem erfindungsgemäßen bismuthaltigen Katalysator ein Polyol als erste Komponente sowie ein Polyisocyanat als zweite Komponente.

Als "zweikomponentig" wird eine Zusammensetzung bezeichnet, bei welcher die Bestandteile derselben in zwei verschiedenen Komponenten vorliegen, welche in voneinander getrennten Gebinden gelagert werden, und welche jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und/oder erhöhter Temperatur abläuft oder vervollständigt wird.

Mit "Poly" beginnende Substanznamen, wie Polyol oder Polyisocyanat, bezeichnen Substanzen, die formal zwei oder mehr der in ihrem Namen vorkommenden funktionellen Gruppen pro Molekül enthalten.

Der Begriff "Polyisocyanat" umfasst Verbindungen mit zwei oder mehr Isocyanatgruppen, unabhängig davon, ob es sich dabei um monomere Diisocyanate, oligomere Polyisocyanate oder Isocyanatgruppen aufweisende Polymere handelt.

Als Polyisocyanat geeignet ist beispielsweise ein Polyisocyanat in Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise 1,4-Tetramethylen-diisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3-und -1,4-diisocyanat, 1-Methyl-2,4- und -2,6-diisocyanato-cyclohexan und beliebige Gemische dieser Isomeren, 1-Isocyanato-3,3,5-trimethyl-5-isocyanatomethyl-cyclohexan (= Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclohexan, m- und p-Xylylen-diisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate, wie 3,6-Bis-(9-isocyanatononyl)-4,5-di-(1-heptenyl)-cyclohexen (Dimeryldiisocyanat), a,a,a',a',a",a"-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI),

1,3,5-Tris-(isocyanatomethyl)-benzol, Tris-(4-isocyanatophenyl)-methan und Tris-(4-isocyanatophenyl)-thiophosphat.

Bevorzugte Polyisocyanate sind handelsübliche Diisocyanate. Besonders bevorzugt sind HDI, IPDI, TDI und MDI sowie Oligomere von Diisocyanaten und Isocyanatgruppen aufweisende Polyurethanpolymere (NCO-Prepolymere).

Als Polyole können beispielsweise die folgenden handelsüblichen Polyole oder Mischungen davon eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
  Besonders geeignet sind Polyoxyalkylendiole oder Polyoxyalkylentriole, insbesondere Polyoxyethylen- und Polyoxypropylendi- und -triole. Speziell geeignet sind Polyoxyalkylendiole und -triole mit einem Ungesättigtheitsgrad tiefer als 0,02 mEq/g und mit einem Molekulargewicht im Bereich von 1.000 - 30.000 g/mol, sowie Polyoxypropylendiole und -triole mit einem Molekulargewicht von 400 - 8.000 g/mol.
  Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.

- Styrol-Acrylnitril- oder Acrylnitril-Methylmethacrylat-gepfropfte Polyetherpolyole.

- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.
  Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Capro-lacton und Startern wie den vorgenannten zwei- oder dreiwertigen Alkoholen.

- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.

- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.

- Polyacrylat- und Polymethacrylatpolyole.

- Polyhydroxyfunktionelle Fette und Öle, beispielsweise natürliche Fette und Öle, insbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschlie-βender Ringöffnung mit Carbonsäuren bzw.

Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschlie-βender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.

- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/Butadien-Copolymere, wie sie beispielsweise aus Epoxiden oder Aminoalkoholen und carboxylterminierten Acrylonitril/Butadien-Copolymeren hergestellt werden können; sowie hydrierte polyhydroxyfunktionelle Polymere oder Copolymere von Dienen.

Die genannten Polyole weisen bevorzugt ein mittleres Molekulargewicht von 250 - 30.000 g/mol, insbesondere von 400 - 20.000 g/mol, auf und weisen ferner bevorzugt eine mittlere OH-Funktionalität im Bereich von 1,6 bis 3 auf.

Unter "Molekulargewicht" versteht man bei Oligomeren oder Polymeren stets das Molekulargewichtsmittel $M_n$.

Besonders bevorzugt ist der Einsatz von Polyetherpolyolen, vorzugsweise Polypropylenpolyolen und Polyethylen-polypropylen-Mischpolyolen, sowie Polyesterpolyolen und Polycarbonatpolyolen.

Der erfindungsgemäße bismuthaltige Katalysator liegt vorzugsweise in der ersten Komponente vor, was den Vorteil hat, dass das auf katalytisch wirkende Verbindungen empfindliche Polyisocyanat in der zweiten Komponente in seiner Lagerstabilität (Haltbarkeit) nicht beeinträchtigt wird.

Der erfindungsgemäße bismuthaltige Katalysator kann als alleiniger Katalysator oder auch zusammen mit anderen Katalysatoren, wie beispielsweise Bismut-, Zinn- oder Zirkoniumverbindungen oder tertiären Aminen, eingesetzt werden.

Der erfindungsgemäße bismuthaltige Katalysator kann gegebenenfalls weitere üblicherweise eingesetzte Hilfs- und Zusatzstoffe, beispielsweise Pigmente, Weichmacher bzw. Verdünner, Härter, Vernetzer, Kettenverlängerer, weitere Katalysatoren, Haftmittel, Stabilisatoren, Rheologiehilfsmittel und Trocknungsmittel, etc. enthalten.

Der erfindungsgemäße bismuthaltige Katalysator, betrachtet als Menge elementares Bismut, liegt in der erfindungsgemäßen zweikomponentigen Polyurethan-Zusammensetzung bevorzugt in einer Menge von 0,0002 bis 1 Gew.-%, besonders bevorzugt in einer Menge von 0,001 bis 0,2 Gew.-%, und ganz besonders bevorzugt in einer Menge von 0,002 bis 0,1 Gew.-%, bezogen auf das Gewicht der Zusammensetzung, vor. Zu hohe Mengen führen dazu, dass die Offenzeit bzw. Verarbeitungszeit der Zusammensetzung zu kurz ist, während der Einsatz zu geringer Mengen den Nachteil hat, dass die Zusammensetzung zu schwach katalysiert ist und damit zu langsam, unvollständig und/oder fehlerhaft aushärtet. In der erfindungsgemäßen zweikomponentigen Polyurethan-Zusammensetzung macht der erfindungsgemäße bismuthaltige Katalysator 0,001 bis 5, bevorzugt 0,005 bis 1, und besonders bevorzugt 0,01 bis 0,5 mmol-Equivalente Bismutatome auf 100 g der Zusammensetzung aus.

Wie bereits vorstehend erwähnt, ist der erfindungsgemäße bismuthaltige Katalysator hinsichtlich der Urethanisierungsreaktion vergleichsweise aktiv sowie auch vergleichsweise selektiv. So zeichnet sich der erfindungsgemäße bismuthaltige Katalysator durch eine hohe katalytische Aktivität auch bei längerer Lagerung aus. Die Aushärtung der erfindungsgemäßen zweikomponentigen Polyurethan-Zusammensetzung erfolgt im Allgemeinen rasch. Die Selektivität des erfindungsgemäßen bismuthaltigen Katalysators leidet nicht unter der erhöhten Aktivität; die Aushärtung erfolgt ohne Bildung von Blasen, selbst bei ungünstigen Bedingungen, wie hoher Temperatur, hoher Umgebungsfeuchte bzw. hohem Restwassergehalt der Zusammensetzung sowie bei Einsatz von Polyolen mit sekundären OH-Gruppen oder hydrophilen Polyolen. Der erfindungsgemäße bismuthaltige Katalysator ist thermisch und hydrolytisch vergleichsweise stabil, zersetzt sich selbst in einem restwasserhaltigen Polyol nur langsam und behält somit seine katalytische Aktivität auch bei längerer Lagerdauer. Dennoch führt der Einsatz des erfindungsgemäßen bismuthaltigen Katalysators zu guter Stabilität der ausgehärteten Polyurethan-Zusammensetzung unter thermischer Belastung. Der erfindungsgemäße bismuthaltige Katalysator ist ferner bei Raumtemperatur flüssig und/oder in Weichmachern oder Polyolen gut löslich und lässt sich somit in bei Raumtemperatur härtenden Systemen auf einfache Weise und insbesondere ohne Verwendung von flüchtigen organischen Lösungsmitteln (VOC) einsetzen. Schließlich ist der erfindungsgemäße bismuthaltige Katalysator farblos und verfärbt auch unter Lichteinfluss nicht, so dass er auch in unpigmentierten oder hellfarbigen Produkten ohne Beimischung eingesetzt werden kann. Die mit dem erfindungsgemäßen bismuthaltigen Katalysator ausgehärteten Polyurethan-Zusammensetzungen zeigen trotz dessen Hydrolysestabilität eine gute thermische Beständigkeit und neigen nicht stärker zu Verfärbung als solche, welche mit Bismut(III)-carboxylaten ausgehärtet wurden.

Die erfindungsgemäße zweikomponentige Polyurethan-Zusammensetzung kann in vielen Bereichen eingesetzt werden, beispielsweise als Vergussmasse, Dichtstoff, Klebstoff, Belag, Beschichtung, Lack, Voranstrich, Hartschaum, Weichschaum, Formteil, Elastomer, Faser, Folie oder Membran für Bau- und Industrieanwendungen, beispielsweise als Elektrovergussmasse, Spachtelmasse, Nahtabdichtung, Hohlraumversiegelung, Fugendichtstoff, Montageklebstoff, Karosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag und -beschichtung, Balkon-und Dachbeschichtung, Betonschutzbeschichtung, Parkhausbeschichtung, Rohrbeschichtung, Korrosionsschutzbeschichtung, Textilbeschichtung, Holzlack, Dekorationslack, Primer, Möbelschaum, Polsterschaum, Filterschaum, Isolationsschaum, Schalldämmschaum, Abdichtungsschaum, Verpackungsschaum, Karosserieschaum, Modellbauplatte, Dämpfungselement, Dichtungselement, Reifen, Rollen, Lager, Walze, Förderband, Gummifaden, Schuhsohle, Gehäuse, Fensterprofil, Implantat, Schaumgummi, etc.

Bevorzugte Anwendungsgebiete sind Vergussmassen, Dichtstoffe, Klebstoffe, Beläge, Beschichtungen, Lacke, Voranstriche, Formteile und Elastomere für Bau- und Industrieanwendungen.

Der erfindungsgemäße bismuthaltige Katalysator kann auch in einkomponentigen Polyurethan-Zusammensetzungen eingesetzt werden. Diese umfassen neben dem erfindungsgemäßen bismuthaltige Katalysator mindestens ein Polyurethanprepolymer mit Isocyanatendgruppen, welches aus mindestens einem Polyisocyanat und mindestens einem Polyol hergestellt ist. Das Polyurethanprepolymer wird in üblicher Weise hergestellt, beispielsweise wie in der EP 1 408 062 A1 beschrieben. Die für die Prepolymerherstellung eingesetzten Polyole sind solche wie in der EP 1 408 062 und vorstehend beschrieben. Gleiches gilt für die zur Herstellung der Polyurethanprepolymere verwendeten Polyisocyanate.

In der erfindungsgemäßen einkomponentigen Polyurethan-Zusammensetzung macht der erfindungsgemäße bismuthaltige Katalysator 0,02 bis 5, bevorzugt 0,1 bis 2,5, und besonders bevorzugt 0,2 bis 1 mmol-Equivalente Bismutatome auf 100 g der Zusammensetzung aus.

Einkomponentige Polyurethan-Zusammensetzungen enthaltend einen erfindungsgemäßen bismuthaltigen Katalysator weisen typischerweise gleiche bzw. ähnliche Vorteile wie die vorstehend beschriebenen zweikomponentigen Polyurethan-Zusammensetzungen auf, insbesondere vergleichsweise gute Lagerstabilität und Hautbildungszeiten.

Die Anwendungsgebiete der erfindungsgemäßen einkomponentigen Polyurethan-Zusammensetzung entsprechen denjenigen der vorstehend im Zusammenhang mit den zweikomponentigen Polyurethan-Zusammensetzungen erwähnten Anwendungen.

Außer in einkomponentigen und zweikomponentigen Polyurethan-Zusammensetzungen kann der erfindungsgemäße bismuthaltige Katalysator als Katalysator oder Co-Katalysator auch in anderen härtbaren Massen eingesetzt werden, beispielsweise, in Epoxidharzen, Acrylaten und Silikonen.

**Beispiele**

Beschreibung der Messmethoden

Infrarotspektren wurden auf einem FT-IR Gerät 1600 von Perkin-Elmer gemessen (horizontale ATR-Messeinheit mit ZnSe-Kristall; Messfenster 4000-650 $cm^{-1}$). Flüssige Proben wurden unverdünnt als Filme aufgetragen, feste Proben wurden in $CH_2Cl_2$ gelöst. Die Absorptionsbanden sind in Wellenzahlen ($cm^{-1}$) angegeben.

$^1$H-NMR-Spektren wurden auf einem Spektrometer des Typs Bruker DPX-300 bei 300,13 MHz gemessen; die chemischen Verschiebungen $\delta$ sind angegeben in ppm relativ zu Tetramethylsilan (TMS). Echte und Pseudo-Kopplungsmuster wurden nicht unterschieden.

Die Viskosität wurde auf einem thermostatisierten Kegel-Platten-Viskosimeter Physica MCR 300 (Kegeldurchmesser 20 mm, Kegelwinkel 1°, Kegelspitze-Platten-Abstand 0,05 mm, Schergeschwindigkeit 0,1 bis 100 $s^{-1}$) gemessen.

UV-vis-Spektren von in Dichlormethan gelösten Proben (40 mg/l) in 1 cm Quarzküvetten wurden auf einem Spektrometer des Typs Varian Cary 50 im Wellenlängenbereich 800 - 200 nm gemessen. Angegeben sind die Extinktionsmaxima $\lambda_{max}$ in nm und in Klammern die zugehörigen Extinktionskoeffizienten $\varepsilon$ in $l \cdot g^{-1} \cdot cm^{-1}$.

Herstellung der Polyurethan-Katalysatoren

**Allgemeine Herstellvorschrift A**

In einem Rundkolben wurden kommerzielles Bismut(III)-tris(neodecanoat) und ein 1,3-Ketoamid vermischt und die Mischung unter Rühren während 2 Stunden auf 80 °C erwärmt. Darauf wurde das Reaktionsgemisch auf Raumtemperatur gekühlt.

**Beispiel 1: Katalysator Bi1**

Gemäß der allgemeinen Herstellvorschrift A wurden 7,75 g Coscat® 83 (Bismut(III)-tris(neodecanoat) in Neodecansäure; 16% Bi; von Erbslöh) und 2,85 g N,N-Diethyl-3-oxobutanamid umgesetzt. Man erhielt ein schwach gelbes Öl.
FT-IR: 2957, 2933, 2873, 1722, 1698, 1636, 1606, 1545, 1462, 1381, 1361, 1313, 1272, 1217, 1162, 1098, 1080, 942, 907, 872, 826, 789.
UV-vis: 258 (0,5). (vgl. Coscat® 83: 267(0,3).)

**Beispiel 2: Katalysator Bi2**

Gemäß der allgemeinen Herstellvorschrift A wurden 4,29 g Coscat® 83 (16% Bi; von Erbslöh) und 3,11 g N,N-Diethyl-3-oxobutanamid umgesetzt. Man erhielt ein schwach gelbes Öl.
FT-IR: 2958, 2931, 2872, 1722, 1636, 1605, 1460, 1381, 1360, 1313, 1272, 1217, 1148, 1098, 1080, 942, 921, 826, 774, 730.

**Beispiel 3: Katalysator Bi3**

Gemäß der allgemeinen Herstellvorschrift A wurden 6,62 g NeoBi 200BA (Bismut(III)-tris(neodecanoat) in Neodecansäure; 20% Bi; von Shepherd) und 5,98 g N,N-Diethyl-3-oxobutanamid umgesetzt. Man erhielt ein schwach gelbes Öl.
FT-IR: 2960, 2931, 2874, 1720, 1635, 1593, 1490, 1461, 1390, 1359, 1312, 1272, 1220, 1199, 1148, 1098, 1080, 1007, 942, 919, 826, 774, 728.

**Beispiel 4: Katalysator Bi4**

Gemäß der allgemeinen Herstellvorschrift A wurden 1,40 g Coscat® 83 (16% Bi; von Erbslöh) und 1,38 g N,N-Dibutyl-3-oxobutanamid umgesetzt. Man erhielt ein schwach gelbes Öl.
FT-IR: 2957, 2930, 2872, 1721, 1635, 1605, 1490, 1461, 1362, 1293, 1226, 1159, 931, 773, 732.

**Beispiel 5: Katalysator Bi5**

Gemäß der allgemeinen Herstellvorschrift A wurden 2,63 g Coscat® 83 (16% Bi; von Erbslöh) und 3,09 g N,N-Dibutyl-3-oxoheptanamid umgesetzt. Man erhielt ein schwach gelbes Öl.
FT-IR: 2956, 2930, 2872, 1717, 1630, 1607, 1489, 1464, 1378, 1292, 1255, 1222, 1146, 932, 817, 775, 731.

**Beispiel 6: Katalysator Bi6**

Gemäß der allgemeinen Herstellvorschrift A wurden 4,33 g Coscat® 83 (16% Bi; von Erbslöh) und 3,42 g N,N-Bis(2-ethylhexyl)-3-oxobutanamid umgesetzt. Man erhielt ein fast farbloses Öl.
FT-IR: 2957, 2927, 2872, 1698, 1632, 1602, 1546, 1461, 1380, 1359, 1232, 1159, 1006, 934, 818, 773, 728.

**Beispiel 7: Katalysator Bi7**

Gemäß der allgemeinen Herstellvorschrift A wurden 4,16 g Coscat® 83 (16% Bi; von Erbslöh) und 3,51 g N,N-Bis(2-ethylhexyl)-2-oxocyclopentancarboxamid umgesetzt. Man erhielt ein schwach gelbes Öl.
FT-IR: 2957, 2928, 2872, 1740, 1698, 1640, 1606, 1546, 1536, 1460, 1379, 1216, 1149, 1108, 1003, 935, 904, 872, 833, 766, 728.

**Beispiel 8: Katalysator Bi8**

Gemäß der allgemeinen Herstellvorschrift A wurden 4,53 g Coscat® 83 (16% Bi; von Erbslöh) und 3,00 g N,N-Dibutyl-3-oxo-3-phenylpropanamid umgesetzt. Man erhielt ein hellgelbes Öl.
FT-IR: 2957, 2931, 2871, 1736, 1695, 1599, 1575, 1484, 1466, 1366, 1292, 1215, 1153, 1085, 1000, 942, 907, 874,817, 764, 716, 688.

**Vergleichsbeispiel: Katalysator Bi9**

In einem Rundkolben wurden 0,88 g Coscat® 83 (16% Bi; von Erbslöh) und 0,21 g 2,4-Pentandion vermischt

und die Mischung unter Rühren während 2 Stunden auf 80 °C erwärmt. Darauf wurde das Reaktionsgemisch auf Raumtemperatur gekühlt. Man erhielt ein farbloses Öl.

**Vergleichsbeispiel: Katalysator Bi10**

In einem Rundkolben wurden 1,40 g Coscat® 83 (16% Bi; von Erbslöh) und 0,66 g 2,4-Pentandion vermischt und die Mischung unter Rühren während 2 Stunden auf 80 °C erwärmt. Darauf wurde das Reaktionsgemisch auf Raumtemperatur gekühlt. Man erhielt ein farbloses Öl.

**Vergleichsbeispiel: Katalysator Bi11**

In einem Rundkolben wurden 1,10 g Coscat® 83 (16% Bi; von Erbslöh) und 0,34 g Ethylacetoacetat vermischt und die Mischung unter Rühren während 2 Stunden auf 80 °C erwärmt. Darauf wurde das Reaktionsgemisch auf Raumtemperatur gekühlt. Man erhielt ein leicht rosafarbenes Öl.

**Vergleichsbeispiel: Katalysator Bi12**

In einem Rundkolben wurden 1,20 g Coscat® 83 (16% Bi; von Erbslöh) und 0,73 g Ethylacetoacetat vermischt und die Mischung unter Rühren während 2 Stunden auf 80 °C erwärmt. Darauf wurde das Reaktionsgemisch auf Raumtemperatur gekühlt. Man erhielt ein leicht rosafarbenes Öl.

**Vergleichsbeispiel: Katalysator Bi13**

In einem Rundkolben wurden 1,25 g Coscat® 83 (16% Bi; von Erbslöh) und eine Lösung von 0,44 g 8-Hydroxychinolin in 3,27 g Diisodecylphthalat vermischt und die Mischung unter Rühren während 2 Stunden auf 80 °C erwärmt. Darauf wurde das Reaktionsgemisch auf Raumtemperatur gekühlt. Man erhielt eine tiefgelbe Suspension.

**Vergleichsbeispiel: Katalysator Bi14**

In einem Rundkolben wurden 1,26 g Coscat® 83 (16% Bi; von Erbslöh) und eine Lösung von 0,40 g Salicylsäure in 3,49 g Diisodecylphthalat vermischt und die Mischung unter Rühren während 2 Stunden auf 80 °C erwärmt. Darauf wurde das Reaktionsgemisch auf Raumtemperatur gekühlt. Man erhielt eine fast farblose Flüssigkeit.

<u>Zweikomponentige Polyurethan-Zusammensetzungen</u>

**Beispiele 9 bis 10 und Vergleichsbeispiele V1 bis V5**

Zur Herstellung der ersten Komponente wurden für jedes Beispiel ein Polyethertriol (Voranol® CP 4755, von Dow) und ein Katalysator gemäß Tabelle 1 in einem Zentrifugalmischer (SpeedMixer™ DAC 150, FlackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur® CD-L, von Bayer) als zweite Komponente gemäß Tabelle 1 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 1 auf die gleiche Weise zu einer Polyurethan-Zusammensetzung vermischt.

| Beispiel | **9** | **10** | **V1** | **V2** | **V3** | **V4** | **V5** |
|---|---|---|---|---|---|---|---|
| *Erste Komponente:* | | | | | | | |
| Voranol® CP 4755 | 50 | 50 | 50 | 50 | 50 | 50 | 50 |
| **Katalysator Bi1** | 0,014 | - | - | - | - | - | - |
| **Katalysator Bi2** | - | 0,022 | - | - | - | - | - |
| **Katalysator Bi9** | - | - | 0,013 | - | - | - | - |

(fortgesetzt)

| Beispiel | 9 | 10 | V1 | V2 | V3 | V4 | V5 |
|---|---|---|---|---|---|---|---|
| *Erste Komponente:* | | | | | | | |
| **Katalysator Bi10** | - | - | - | 0,026 | - | - | - |
| **Katalysator Bi11** | - | - | - | - | 0,011 | - | - |
| **Katalysator Bi13** | - | - | - | - | - | 0,040 | - |
| Coscat® 83[a] | - | - | - | - | - | - | 0,020 |
| mmol-Equiv./100g[b] | 0,014 | 0,018 | 0,015 | 0,025 | 0,012 | 0,014 | 0,028 |
| *Zweite Komponente:* | | | | | | | |
| Desmodur® CD-L | 5,10 | 5,10 | 5,10 | 5,10 | 5,10 | 5,10 | 5,10 |

**Tabelle 1: Zweikomponentige Polyurethan-Zusammensetzungen** (Mengen in Gewichtsteilen).
[a] Bismut(III)-tris(neodecanoat) in Neodecansäure (16% Bi, von Erbslöh). [b] mmol-Equivalente Bismutatome des Katalysators auf 100 g der Zusammensetzung.

Die Polyurethan-Zusammensetzungen wurden auf Aspekt, Farbe, Zeit bis zur Klebefreiheit, Blasenbildung sowie Shore A-Härte geprüft, und zwar jeweils sowohl für die Zusammensetzung mit der frisch hergestellten ersten Komponente als auch für die Zusammensetzung mit der während 7 Tagen bei 60 °C gelagerten ersten Komponente. Ausschließlich für die Zusammensetzung mit der frisch hergestellten ersten Komponente wurde weiterhin die mechanischen Eigenschaften im Zugversuch gemessen, und zwar vor und nach verschiedenen Lagerungen zur beschleunigten Alterung der Proben.

Der Aspekt und die Farbe der Zusammensetzung wurden rein optisch beurteilt, wobei der Aspekt als "klar", "trüb" oder inhomogen ("inh.") gewertet wurde.

Zur Bestimmung der Zeit bis zur Klebefreiheit (Hautbildungszeit) wurden die raumtemperaturwarmen Zusammensetzungen in einer Schichtdicke von ca. 3 mm auf Pappkarton aufgetragen und im Normklima ("NK"; 23±1 °C, 50±5% relative Luftfeuchtigkeit) jeweils die Zeit bestimmt, die es dauerte, bis beim leichten Antippen der Oberfläche einer Zusammensetzung mittels einer Pipette aus LDPE erstmals keine Rückstände auf der Pipette mehr zurückblieben.

Die Blasenbildung wurde optisch beurteilt anhand der Menge ("viele", "einige", "keine") an Gasblasen, die in der für die Bestimmung der Hautbildungszeit angesetzten Zusammensetzung während deren Aushärtung auftrat.

Die Shore A-Härte wurde bestimmt nach DIN 53505 an während 7 Tagen im Normklima ausgehärteten Prüfkörpern.

Zur Bestimmung der mechanischen Eigenschaften im Zugversuch wurde von den Zusammensetzungen Filme von ca. 3 mm Dicke hergestellt, indem die Zusammensetzung in eine plane PTFE-Form gegossen und während 7 Tagen im Normklima ausgehärtet wurde. Es wurden klebfreie und elastische Filme erhalten. Aus den Filmen wurden Hanteln mit einer Länge von 75 mm, bei einer Steglänge von 30 mm und einer Stegbreite von 4 mm, ausgestanzt und ein Teil davon gemäß DIN EN 53504 bei einer Zuggeschwindigkeit von 200 mm/min auf Zugfestigkeit, Bruchdehnung und E-Modul (bei 0,5 bis 5,0% Dehnung) geprüft. Der verbleibende Teil der Hanteln wurde während 1 Tag bei 100 °C im Umluftofen, bzw. während 10 Tagen unter "Kataplasma" (40 °C und 100% relative Luftfeuchtigkeit), bzw. während 10 Tagen unter "Kataplasma" sowie 1 Tag bei 100 °C, gelagert, darauf jeweils während einem Tag im Normklima gehalten und gemäß DIN EN 53504 wie beschrieben geprüft.

Die Ergebnisse dieser Prüfungen sind in der Tabelle 2 aufgeführt.

| Beispiel | 9 | 10 | V1 | V2 | V3 | V4 | V5 |
|---|---|---|---|---|---|---|---|
| *Zusammensetzung mit frisch hergestellter erster Komponente:* | | | | | | | |
| Aspekt | klar | klar | klar | klar | klar | inh. | klar |
| Farbe | farblos | farblos | farblos | farblos | farblos | gelb | farblos |
| Hautbildungszeit (min.) | 8 | 8 | 3 | 10 | 10 | 300 | 3 |
| Shore A-Härte | 43 | 51 | 46 | 47 | 47 | 48 | 44 |
| Blasenbildung | keine | keine | keine | keine | keine | keine | keine |
| Zugfestigkeit (MPa): 7d/NK | 0,70 | 0,70 | 0,70 | 0,81 | 0,92 | 0,72 | 0,54 |

(fortgesetzt)

| Beispiel | 9 | 10 | V1 | V2 | V3 | V4 | V5 |
|---|---|---|---|---|---|---|---|
| *Zusammensetzung mit frisch hergestellter erster Komponente:* | | | | | | | |
| +10d/Kataplasma | 0,73 | 0,83 | 0,65 | 0,82 | 0,79 | 0,71 | 0,79 |
| +1d/100°C | 0,75 | 0,98 | 0,87 | 0,87 | 0,85 | 0,68 | 0,73 |
| +10d/Kataplasma+1d/100°C | 0,85 | 0,86 | 0,79 | 0,77 | 0,93 | 0,67 | 0,73 |
| Bruchdehnung (%): 7d/NK | 49 | 47 | 51 | 74 | 86 | 122 | 42 |
| +10d/Kataplasma | 59 | 60 | 50 | 75 | 66 | 122 | 73 |
| +1d/100°C | 73 | 95 | 80 | 93 | 93 | 101 | 72 |
| +10d/Kataplasma+1d/100°C | 82 | 78 | 72 | 81 | 95 | 93 | 74 |
| E-Modul (MPa): 7d/NK | 1,81 | 2,00 | 1,72 | 1,61 | 1,68 | 0,74 | 1,46 |
| +10d/Kataplasma | 1,80 | 1,97 | 1,69 | 1,66 | 1,77 | 0,97 | 1,56 |
| +1d/100°C | 1,48 | 1,82 | 1,72 | 1,50 | 1,64 | 1,03 | 1,49 |
| +10d/Kataplasma+1d/100°C | 1,59 | 1,74 | 1,72 | 1,42 | 1,63 | 1,07 | 1,41 |
| *Zusammensetzung mit gelagerter erster Komponente:* | | | | | | | |
| Aspekt | klar | klar | klar | klar | klar | inh. | klar |
| Farbe | farblos | farblos | farblos | farblos | farblos | tiefgelb | farblos |
| Hautbildungszeit (min.) | 15 | 12 | 20 | 82 | 205 | 12 | 45 |
| Shore A-Härte | 47 | 47 | 44 | 46 | 47 | 48 | 45 |
| Blasenbildung | keine | keine | keine | einige | keine | keine | einige |
| **Tabelle 2: Eigenschaften der zweikomponentigen Polyurethan-Zusammensetzungen** | | | | | | | |

Aus der Tabelle 2 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und blasenfrei zu einem Material mit vergleichsweise hoher Festigkeit und guter Beständigkeit aushärten.

**Beispiele 11 bis 12 und Vergleichsbeispiele V6 bis V10**

Zur Herstellung der ersten Komponente wurden für jedes Beispiel ein Polyethertriol (Voranol® CP 4755, von Dow), ein Polyetherdiol (Acclaim® 4200, von Bayer) und ein

Katalysator gemäß Tabelle 3 in einem Zentrifugalmischer (SpeedMixer™ DAC 150, FlackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60°C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur® CD-L, von Bayer) als zweite Komponente gemäß Tabelle 3 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 3 auf die gleiche Weise zu einer Polyurethan-Zusammensetzung vermischt.

| Beispiel | 11 | 12 | V6 | V7 | V8 | V9 | V10 |
|---|---|---|---|---|---|---|---|
| *Erste Komponente:* | | | | | | | |
| Voranol® CP 4755 | 33,3 | 33,3 | 33,3 | 33,3 | 33,3 | 33,3 | 33,3 |
| Acclaim® 4200 | 16,7 | 16,7 | 16,7 | 16,7 | 16,7 | 16,7 | 16,7 |
| Katalysator **Bi1** | 0,022 | - | - | - | - | - | - |
| Katalysator **Bi3** | - | 0,030 | - | - | - | - | - |
| Katalysator **Bi9** | - | - | 0,017 | - | - | - | - |

(fortgesetzt)

| Beispiel | **11** | **12** | **V6** | **V7** | **V8** | **V9** | **V10** |
|---|---|---|---|---|---|---|---|
| *Erste Komponente:* | | | | | | | |
| Katalysator **Bi11** | - | - | - | 0,014 | - | - | - |
| Katalysator **Bi12** | - | - | - | - | 0,025 | - | - |
| Katalysator **Bi14** | - | - | - | - | - | 0,040 | - |
| Coscat® 83[a] | - | - | - | - | - | - | 0,020 |
| mmol-Equiv./100g[b] | 0,022 | 0,027 | 0,019 | 0,015 | 0,022 | 0,014 | 0,028 |
| *Zweite Komponente:* | | | | | | | |
| Desmodur® CD-L | 5,00 | 5,00 | 5,00 | 5,00 | 5,00 | 5,00 | 5,00 |

**Tabelle 3: Zweikomponentige Polyurethan-Zusammensetzungen** (Mengen in Gewichtsteilen).
[a] Bismut(III)-tris(neodecanoat) in Neodecansäure (16% Bi, von Erbslöh).[b] mmol-Equivalente Bismutatome des Katalysators auf 100 g der Zusammensetzung.

Die Polyurethan-Zusammensetzungen wurden wie für Beispiel 9 beschrieben auf Aspekt, Zeit bis zur Klebefreiheit, Blasenbildung sowie die mechanischen Eigenschaften im Zugversuch geprüft, und zwar jeweils nur für die Zusammensetzung mit der frisch hergestellten ersten Komponente.

Die Ergebnisse dieser Prüfungen sind in der Tabelle 4 aufgeführt.

| Beispiel | **11** | **12** | **V6** | **V7** | **V8** | **V9** | **V10** |
|---|---|---|---|---|---|---|---|
| *Zusammensetzung mit frisch hergestellter erster Komponente:* | | | | | | | |
| Aspekt | klar | klar | klar | klar | klar | klar | klar |
| Hautbildungszeit (min.) | 15 | 50 | 30 | 40 | 15 | 180 | 25 |
| Blasenbildung | keine | keine | keine | keine | keine | einige | keine |
| Zugfestigkeit (MPa): 7d/NK | 0,91 | 0,71 | 0,96 | 0,83 | 0,73 | 0,64 | 0,92 |
| +10d/Kataplasma | 0,81 | 0,80 | 0,74 | 0,81 | 0,81 | 0,65 | 0,92 |
| +1d/100°C | 0,75 | 0,75 | 0,86 | 0,78 | 0,71 | 0,67 | 0,99 |
| +10d/Kataplasma+1d/100°C | 0,83 | 0,81 | 0,74 | 0,81 | 0,68 | 0,72 | 0,98 |
| Bruchdehnung (%): 7d/NK | 105 | 104 | 127 | 106 | 90 | 158 | 97 |
| +10d/Kataplasma | 95 | 103 | 94 | 122 | 105 | 151 | 98 |
| +1d/100°C | 122 | 139 | 152 | 141 | 113 | 158 | 118 |
| +10d/Kataplasma+1d/100°C | 121 | 127 | 104 | 121 | 97 | 187 | 103 |
| E-Modul (MPa): 7d/NK | 1,48 | 1,04 | 1,30 | 1,37 | 1,23 | 0,54 | 1,67 |
| +10d/Kataplasma | 1,44 | 1,22 | 1,25 | 0,98 | 1,33 | 0,55 | 1,58 |
| +1d/100°C | 1,12 | 0,97 | 0,78 | 1,00 | 1,03 | 0,75 | 1,46 |
| +10d/Kataplasma+1d/100°C | 1,23 | 0,93 | 0,94 | 0,81 | 1,06 | 0,53 | 1,60 |
| *Zusammensetzung mit gelagerter erster Komponente:* | | | | | | | |
| Aspekt | klar | klar | inh. | klar | klar | klar | klar |
| Hautbildungszeit (min.) | 25 | 55 | 120 | 270 | 35 | 210 | >240 |
| Blasenbildung | keine | keine | keine | keine | keine | einige | viele |

**Tabelle 4: Eigenschaften der zweikomponentigen Polyurethan-Zusammensetzungen**

Aus der Tabelle 4 ist ersichtlich, dass die zweikomponentigen Polyurethanzusammensetzungen mit den erfindungsgemäßen Katalysatoren klare, homogene Mischungen darstellen, welche vergleichsweise kurze Hautbildungszeiten aufweisen und blasenfrei zu einem Material mit vergleichsweise hoher Festigkeit und guter Beständigkeit aushärten.

**Beispiele 13 bis 19**

Wie für Beispiel 9 beschrieben wurden zur Herstellung der ersten Komponente jeweils ein Polyethertriol (Voranol® CP 4755, von Dow) und ein Katalysator gemäß Tabelle 5 vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die für Beispiel 9 beschriebene Weise mit einem modifizierten, bei Raumtemperatur flüssigen Diphenylmethandiisocyanat (Desmodur® CD-L, von Bayer) als zweite Komponente gemäß Tabelle 5 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 5 auf die gleiche Weise zu einer Polyurethan-Zusammensetzung vermischt.

Die Polyurethan-Zusammensetzungen wurden wie für Beispiel 9 beschrieben auf Aspekt, Zeit bis zur Klebefreiheit, Blasenbildung sowie Shore A-Härte geprüft.

Die Ergebnisse dieser Prüfungen sind in der Tabelle 6 aufgeführt.

| Beispiel | **13** | **14** | **15** | **16** | **17** | **18** | **19** |
|---|---|---|---|---|---|---|---|
| *Erste Komponente:* | | | | | | | |
| Voranol® CP 4755 | 30 | 30 | 30 | 30 | 30 | 30 | 30 |
| **Katalysator Bi1** | 0,075 | - | - | - | - | - | - |
| **Katalysator Bi2** | - | 0,055 | 0,025 | - | - | - | - |
| **Katalysator Bi3** | - | - | - | 0,039 | 0,029 | - | - |
| **Katalysator Bi4** | - | - | - | - | - | 0,027 | - |
| **Katalysator Bi5** | - | - | - | - | - | - | 0,028 |
| mmol-Equiv./100g[a] | 0,127 | 0,074 | 0,033 | 0,059 | 0,044 | 0,031 | 0,030 |
| *Zweite Komponente:* | | | | | | | |
| Desmodur® CD-L | 3,10 | 3,10 | 3,10 | 3,10 | 3,10 | 3,10 | 3,10 |

**Tabelle 5: Zweikomponentige Polyurethan-Zusammensetzungen** (Mengen in Gewichtsteilen).
[a] mmol-Equivalente Bismutatome des Katalysators auf 100 g der Zusammensetzung.

| Beispiel | **13** | **14** | **15** | **16** | **17** | **18** | **19** |
|---|---|---|---|---|---|---|---|
| *Zusammensetzung mit frisch hergestellter erster Komponente:* | | | | | | | |
| Aspekt | klar | klar | klar | klar | klar | klar | klar |
| Hautbildungszeit (min.) | 3 | 7 | 26 | 5 | 2 | 2 | 1 |
| Shore A-Härte | 42 | 44 | 43 | 51 | 46 | 44 | 48 |
| Blasenbildung | keine | keine | keine | keine | keine | keine | keine |
| *Zusammensetzung mit gelagerter erster Komponente:* | | | | | | | |
| Aspekt | klar | klar | klar | klar | klar | klar | klar |
| Hautbildungszeit (min.) | 11 | 15 | 55 | 7 | 5 | 6 | 3 |
| Shore A-Härte | 45 | 43 | 47 | 50 | 49 | 48 | 48 |
| Blasenbildung | keine | keine | keine | keine | keine | keine | keine |

**Tabelle 6: Eigenschaften der zweikomponentigen Polyurethan-Zusammensetzungen**

Aus der Tabelle 6 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und weitgehend blasenfrei zu einem Material mit guter Shore A-Härte aushärten.

Einkomponentige Polyurethan-Zusammensetzungen

**Beispiele 20 bis 23 und Vergleichsbeispiele V11 und V12:**

In einem Polypropylenbecher mit Schraubverschluss wurde für jedes Beispiel das Polyurethanpolymer ***P1***, dessen Herstellung nachfolgend beschrieben wird, mittels eines Zentrifugalmischers (SpeedMixer™ DAC 150, FlackTek Inc.; 1 min. bei 2500 U/min) mit einem Katalysator zu einer homogenen Masse vermischt und die so erhaltene Masse sofort in eine innenlackierte Aluminiumtube abgefüllt und diese luftdicht verschlossen.

Das Polyurethanpolymer ***P1*** wurde wie folgt hergestellt:

1300 g Polyoxypropylen-Diol (Acclaim® 4200 N, von Bayer; OH-Zahl 28,5 mg KOH/g), 2600 g Polyoxypropylenpolyoxyethylen-Triol (Caradol® MD34-02, von Shell; OH-Zahl 35,0 mg KOH/g), 600 g 4,4'-Methylendiphenyldiisocyanat (4,4'-MDI; Desmodur® 44 MC L, von Bayer) und 500 g Diisodecylphthalat (DIDP; Palatinol® Z, von BASF) wurden nach bekanntem Verfahren bei 80 °C zu einem NCO-terminierten Polyurethanpolymeren mit einem Gehalt an freien Isocyanat-Gruppen von 2,05 Gewichts-% umgesetzt.

Die so erhaltenen Zusammensetzungen wurden auf Lagerstabilität sowie auf Aushärtungsgeschwindigkeit geprüft.

Als Maß für die Lagerstabilität wurde die Veränderung der Viskosität während der Lagerung in der Wärme bestimmt. Dazu wurden die Zusammensetzungen in der verschlossenen Tube im Ofen bei 60 °C gelagert und die Viskosität bei 20 °C ein erstes Mal nach 4 Stunden (= "Viskosität frisch") und ein zweites Mal nach 7 Tagen (= "Viskosität gelagert") Lagerdauer gemessen. Die Lagerstabilität ergibt sich aus der prozentualen Zunahme des zweiten Viskositätswerts gegenüber dem ersten. Dazu wird die Viskositätszunahme in % gemäß folgender Formel berechnet:

$$[(\text{Viskosität nach 7 d} / \text{Viskosität nach 4 h}) - 1] \times 100\%.$$

Als Maß für die Aushärtungsgeschwindigkeit wurde die Zeit bis zur Klebefreiheit (Hautbildungszeit) bestimmt, und zwar für die während 4 Stunden bei 60 °C gelagerten Zusammensetzungen (= "HBZ frisch") und für die während 7 Tagen bei 60 °C gelagerten Zusammensetzungen (= "HBZ gelagert"). Dazu wurden die raumtemperaturwarmen Zusammensetzungen in einer Schichtdicke von ca. 3 mm auf Pappkarton aufgetragen und im Normklima ("NK"; 23±1 °C, 50±5% relative Luftfeuchtigkeit) jeweils die Zeit bestimmt, die es dauerte, bis beim leichten Antippen der Oberfläche einer Zusammensetzung mittels einer Pipette aus LDPE erstmals keine Rückstände auf der Pipette mehr zurückblieben.

Die Ergebnisse dieser Prüfungen sind in der Tabelle 7 aufgeführt.

| Beispiel | **20** | **21** | **22** | **23** | **V11** | **V12** |
|---|---|---|---|---|---|---|
| Polyurethanpolymer ***P1*** | 30 | 30 | 30 | 30 | 30 | 30 |
| Katalysator **Beispiel 6** | 0,63 | - | - | - | - | - |
| Katalysator **Beispiel 7** | - | 0,66 | 0,35 | - | - | - |
| Katalysator **Beispiel 8** | - | - | - | 0,59 | - | - |
| Coscat® 83[a] | - | - | - | - | 0,37 | - |
| mmol-Equiv.[b] | 0,9 | 0,9 | 0,5 | 0,9 | 0,9 | - |
| Viskosität frisch (Pa·s) | 71 | 92 | 91 | 88 | 108 | 68 |
| Viskosität gelagert (Pa·s) | 101 | 174 | 133 | 131 | 169 | 97 |
| Viskositätszunahme (%) | 42 | 90 | 45 | 49 | 57 | 42 |
| HBZ frisch (min.) | 50 | 51 | 104 | 47 | 50 | >360 |

(fortgesetzt)

| Beispiel | **20** | **21** | **22** | **23** | **V11** | **V12** |
|---|---|---|---|---|---|---|
| HBZ gelagert (min.) | 53 | 53 | 88 | 55 | 46 | >360 |

**Tabelle** 7: **Einkomponentige Polyurethan-Zusammensetzungen** (Mengen in Gewichtsteilen).
[a] Bismut(III)-tris(neodecanoat) in Neodecansäure (16% Bi, von Erbslöh).[b] mmol-Equivalente Bismutatome des Katalysators auf 100 g der Zusammensetzung.

Aus der Tabelle 7 ist ersichtlich, dass die einkomponentigen Polyurethanzusammensetzungen mit den erfindungsgemäßen Katalysatoren vergleichsweise gute Lagerstabilitäten und Hautbildungszeiten aufweisen.

## Patentansprüche

1. Bismuthaltiger Katalysator, erhältlich durch Umsetzung von mindestens einem Bismut(III)-Salzes oder Bismut (III)-Komplex mit mindestens einem 1,3-Ketoamid der Formel (I)

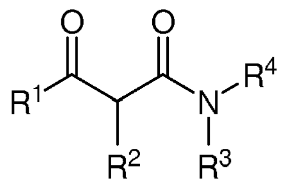

(I),

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

2. Bismuthaltiger Katalysator nach Anspruch 1, wobei der bismuthaltige Katalysator die Formel $Bi(L)_x(Y)_{3-x}$ besitzt, in der x für 1,2 oder 3 steht, y für einen einfach negativen Liganden steht und L für einen Liganden der Formel (I) steht.

3. Bismuthaltiger Katalysator nach Anspruch 1 oder 2, wobei $R^1$ für einen Alkyl-Rest mit 1 bis 4 Kohlenstoffatomen, einen Phenyl-Rest oder zusammen mit $R^2$ für einen zweiwertigen Alkylen-Rest mit 3 bis 4 Kohlenstoffatomen steht.

4. Bismuthaltiger Katalysator nach mindestens einem der vorangegangenen Ansprüche, wobei $R^2$ für einen Wasserstoff-Rest steht.

5. Bismuthaltiger Katalysator nach mindestens einem der vorangegangenen Ansprüche, wobei $R^3$ für einen Wasserstoff-Rest, einen Alkyl-Rest mit 1 bis 8 Kohlenstoffatomen, einen Cycloalkyl-Rest mit 5 bis 6 Kohlenstoffatomen, einen Hydroxyalkyl-Rest mit 1 bis 4 Kohlenstoffatomen, einen Alkylether-Rest mit 1 bis 4 Kohlenstoffatomen, oder zusammen mit $R^4$ für einen zweiwertigen Alkylen-Rest der Formel $-(CH_2)_n-X-(CH_2)_n-$ mit X= O, NR, wobei R ein einwertiger Alkyl-Rest mit 1 bis 4 Kohlenstoffatomen ist, oder S und n= 2 bis 6 steht.

6. Bismuthaltiger Katalysator nach mindestens einem der vorangegangenen Ansprüche, wobei $R^4$ für einen Wasserstoff-Rest, einen Alkyl-Rest mit 1 bis 8 Kohlenstoffatomen, einen Cycloalkyl-Rest mit 5 bis 6 Kohlenstoffatomen, einen Hydroxyalkyl-Rest mit 1 bis 4 Kohlenstoffatomen oder einen Alkylether-Rest mit 1 bis 4 Kohlenstoffatomen steht.

7. Bismuthaltiger Katalysator nach mindestens einem der vorangegangenen Ansprüche, wobei das stöchimetrische Verhältnis zwischen dem Bismut(III)-Salz oder dem Bismut(III)-Komplex und dem 1,3-Ketoamid der Formel (I) im Bereich von etwa 1:0,5 bis 1:20, bevorzugt im Bereich von etwa 1:1 bis 1:10, und besonders bevorzugt im Bereich von etwa 1:3 bis 1:6, liegt.

**8.** Verfahren zur Herstellung des bismuthaltigen Katalysators nach einem der Ansprüche 1 bis 7, wobei mindestens ein Bismut(III)-Salz oder ein Bismut(III)-Komplex mit mindestens einem 1,3 Ketoamid der Formel (I)

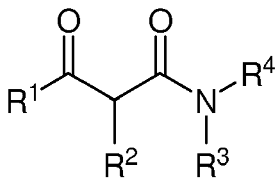

(I)

mit $R^1$, $R^2$, $R^3$ und $R^4$, wie vorstehend definiert, umgesetzt wird.

**9.** Verfahren nach Anspruch 8, wobei als Bismut(III)-Salz oder Bismut(III)-Komplex ein Bismut(III)-carboxylat, vorzugsweise Bismut(III)-neodecanoat oder Bismut(III)-2-ethylhexanoat, eingesetzt wird.

**10.** Verwendung des bismuthaltigen Katalysators nach einem der vorangegangenen Ansprüche 1 bis 7 als Katalysator für härtbare Massen, insbesondere für ein- oder zweikomponentige Polyurethan-Zusammensetzungen.

**11.** Zweikomponentige Polyurethan-Zusammensetzungen, umfassend mindestens ein Polyol als erste Komponente, mindestens ein Polyisocyanat als zweite Komponente und mindestens einen bismuthaltigen Katalysator nach einem der Ansprüche 1 bis 6.

**12.** Zweikomponentige Polyurethan-Zusammensetzung nach Anspruch 11, wobei das Polyol ein Polyetherpolyol und das Polyisocyanat ein Diisocyanat ist.

**13.** Zweikomponentige Polyurethan-Zusammensetzung nach mindestens einem der Ansprüche 11 bis 12, wobei der bismuthaltige Katalysator 0,001 bis 5, bevorzugt 0,005 bis 1, und besonders bevorzugt 0,01 bis 0.5 mmol-Equivalente Bismut auf 100 g der Zusammensetzung ausmacht.

**14.** Zweikomponentige Polyurethan-Zusammensetzung nach mindestens einem der Ansprüche 11 bis 13, wobei der bismuthaltige Katalysator in der ersten Komponente enthalten ist.

**15.** Einkomponentige Polyurethan-Zusammensetzung, umfassend mindestens ein Polyurethanprepolymer mit Isocyanatgruppen, hergestellt aus mindestens einem Polyisocyanat mit mindestens einem Polyol, und mindestens einem bismuthaltigen Katalysator nach einem der Ansprüche 1 bis 7.

**16.** Verwendung der ein- oder zweikomponentigen Polyurethan-Zusammensetzung nach mindestens einem der Ansprüche 11 bis 15 als Vergussmasse, Dichtstoff, Klebstoff, Belag, Beschichtung, Lack, Voranstrich, Formteil, Elastomer für Bau- und Industrieanwendungen.

Europäisches Patentamt
European Patent Office
Office européen des brevets

# EUROPÄISCHER RECHERCHENBERICHT

Nummer der Anmeldung
EP 11 19 3045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2009/050115 A1 (CIBA HOLDING INC [CH]; KOHLI STECK RACHEL [CH]; LORDELOT CAROLINE [FR]) 23. April 2009 (2009-04-23)<br>* Zusammenfassung *<br>* Beispiele 16-19,33-39,56,59 *<br>----- | 1,8,10, 11,15,16 | INV.<br>C07F9/94<br>C07C235/74<br>C07C235/82<br>C08G18/12<br>C09D175/04 |
| A | DATABASE CA [Online]<br>CHEMICAL ABSTRACTS SERVICE, COLUMBUS, OHIO, US;<br>INAGAWA, TAKATOSHI ET AL: "Two-component polyurethane system sealing materials with long pot life",<br>XP002670528,<br>gefunden im STN<br>Database accession no. 2004:950127<br>* Zusammenfassung *<br>& JP 3 587304 B2 (KONISHI CO., LTD., JAPAN) 10. November 2004 (2004-11-10)<br>----- | 1,8,10, 11,15,16 | |
| A,D | EP 1 408 062 A1 (SIKA TECHNOLOGY AG [CH]) 14. April 2004 (2004-04-14)<br>* Zusammenfassung *<br>* Beispiele 1-46 *<br>----- | 1,8,10, 11,15,16 | RECHERCHIERTE SACHGEBIETE (IPC)<br>C07F<br>C07C<br>C08G<br>C09D |

Der vorliegende Recherchenbericht wurde für alle Patentansprüche erstellt

1

| Recherchenort | Abschlußdatum der Recherche | Prüfer |
|---|---|---|
| Den Haag | 29. Februar 2012 | Eberhard, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 11 19 3045

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-02-2012

| Im Recherchenbericht angeführtes Patentdokument | Datum der Veröffentlichung | Mitglied(er) der Patentfamilie | Datum der Veröffentlichung |
|---|---|---|---|
| WO 2009050115 A1 | 23-04-2009 | CN 101874050 A | 27-10-2010 |
| | | EP 2203496 A1 | 07-07-2010 |
| | | JP 2011501774 A | 13-01-2011 |
| | | KR 20100091182 A | 18-08-2010 |
| | | RU 2010119301 A | 27-11-2011 |
| | | US 2010234485 A1 | 16-09-2010 |
| | | WO 2009050115 A1 | 23-04-2009 |
| EP 1408062 A1 | 14-04-2004 | AU 2003285287 A1 | 04-05-2004 |
| | | BR 0315173 A | 23-08-2005 |
| | | CA 2501224 A1 | 22-04-2004 |
| | | CN 1703437 A | 30-11-2005 |
| | | EP 1408062 A1 | 14-04-2004 |
| | | EP 1551895 A1 | 13-07-2005 |
| | | JP 4220467 B2 | 04-02-2009 |
| | | JP 2006502267 A | 19-01-2006 |
| | | MX PA05003678 A | 16-08-2005 |
| | | US 2006180274 A1 | 17-08-2006 |
| | | WO 2004033519 A1 | 22-04-2004 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4584362 A
- WO 2004033519 A
- US 5719229 A
- EP 1408062 A1
- EP 1408062 A